# EUROPÄISCHE PATENTSCHRIFT

(11) **EP 1 237 493 B1**
(45) Veröffentlichungstag und Bekanntmachung des Hinweises auf die Patenterteilung: **14.07.2004**
(21) Anmeldenummer: 00983317.9
(22) Anmeldetag: 13.12.2000
(51) Int. Cl.: A61B 19/00

(54) **VORRICHTUNG ZUR INSTRUMENTEN- UND KNOCHENSEGMENT- SOWIE GEWEBE- UND ORGANNAVIGATION**
DEVICE FOR INSTRUMENT, BONE SEGMENT, TISSUE AND ORGAN NAVIGATION
DISPOSITIF DESTINES AU DEPLACEMENT D'INSTRUMENTS, DE SEGMENTS OSSEUX, DE TISSUS ET D'ORGANES

(30) Priorität: 13.12.1999 DE 19960020
(43) Veröffentlichungstag der Anmeldung: 11.09.2002
(60) Teilanmeldung: 03090311.6
(73) Patentinhaber: LB Medical GmbH, 10719 Berlin (DE)
(72) Erfinder: MARMULLA, Rüdiger, Priv.-Doz- Dr. Dr., 69126 Heidelberg (DE); LÜTH, Tim, Prof. Dr., 12203 Berlin (DE)
(74) Vertreter: Hengelhaupt, Jürgen, Dipl.-Ing.
(86) Internationale Anmeldenummer: PCT/EP2000/012685
(87) Internationale Veröffentlichungsnummer: WO 2001/043654

(56) Entgegenhaltungen:
- EP-A- 0 931 516
- WO-A-99/38449
- DE-A- 19 747 427
- US-A- 5 848 967
- US-A- 5 891 034

## Beschreibung

Die Erfindung betrifft eine Vorrichtung entsprechend dem Oberbegriff des Anspruchs 1.

Um bei einem chirurgischen Eingriff eine Referenzierung zwischen Patientendatensatz und Operationssitus vorzunehmen, werden üblicherweise entweder anatomische Landmarken oder - vor Erstellung eines Bilddatensatzes - am Patienten aufgebrachte Implantate (Knochen- oder Hautmarker) verwendet, die jeweils zugleich mit einem Eingabegerät an der Workstation sowie mit einem Lokalisationssystem am Patienten bezeichnet werden.

Die verallgemeinerte Problemstellung betrifft die Referenzierung zwischen einem Datensatz (der das räumliche Modell eines Körpers geometrisch beschreibt) sowie der realen physikalischen Umwelt in der sich der reale Körper befindet. Für die Referenzierung wird dazu ein dreidimensionaler Lagereferenzkörper am realen Körper verwendet oder angebracht, der aus einem oder mehreren sensorisch räumlich erfaßbaren Elementarkörpern (Markern) besteht, die eine feste geometrische Referenz zum Körperschwerpunkt oder anderen Körperreferenzvolumen selbst definieren. Zur Registrierung erfolgt eine Zuordnung des Lagereferenzkörpers bzw. seiner Elementarkörper im Datenmodell und in der physikalischen Welt.

Abweichend von diesem Verfahren beschreibt das Deutsche Patent DE 19747427 ein Verfahren und eine Vorrichtung, bei der die charakteristische Oberfläche von Knochenstrukturen zur Referenzierung zwischen Datensatz und Operationssitus genutzt wird. Dazu beschreibt DE 197 47 427 eine individuelle Schablone, die 3D-Lokalisationsmarker trägt und auf einem Knochenstück aufgesetzt bzw. festgeschraubt wird.

Nachteil dieser Methode ist, daß die Anfertigung einer individuellen Schablone eine kostenintensive Anfertigung eines CAD-CAM-Modells aus dem Patientendatensatz erforderlich macht. Außerdem ist bei zahlreichen chirurgischen Eingriffen eine breite Freilegung von Knochen zum Aufbringen der individuellen Oberflächenschablone unvermeidlich, wodurch der Eingriff unnötig invasiv wird.

US-A-5 891 034 offenbart eine Vorrichtung gemäß dem Oberbegriff des Anspruchs 1 und hat eine 3D-Marker-Positionserfassungseinheit und einen optischen 3D-Scanner.

Aufgabe der vorliegenden Erfindung ist es, eine Vorrichtung zur Instrumenten- und Knochensegment- sowie Gewebe- und Organnavigation zu schaffen, welche ohne Hilfsmittel wie Schablonen arbeiten und mit einfachen Mitteln eine sichere und reproduzierbare Navigation ermöglichen.

Diese Aufgabe wird mit den Merkmalen des Anspruchs 1 gelöst.

Neben der erheblichen Kostenreduktion kann durch eine optische Erfassung und Referenzierung des OP-Situs über die charakteristische Oberfläche eines Weichteilmantels oder einer Knochenoberfläche der operative Zugangsweg geringer invasiv ausfallen. Außerdem Können die 3D-Lokalisierungsmarker unter Verwendung der vorliegenden

Erfindung bei einer Knochensegmentnavigation unabhängig von einer Schablone - beispielsweise einer Schraube - einzeln an einem Knochensegment befestigt werden, wodurch sich nochmals Möglichkeiten ergeben, einen operativen Eingriff minimal-invasiv zu gestalten.

Eine optische Referenzierung zwischen Datensatz, Operationssitus und 3D-Lokalisationsmarkern erfolgt zudem rascher und exakter als die eingangs erwähnte Referenzierung über anatomische Landmarken und Implantate, weil sich große Oberflächenstrukturen in einem Patientendatensatz (beispielsweise MRT oder CT) insgesamt exakter abbilden und wiedergeben lassen als kleine, einzelne Referenzpunkte.

Ausführungsbeispiele der Erfindung sind in den Zeichnungen dargestellt und werden im folgenden näher beschrieben.

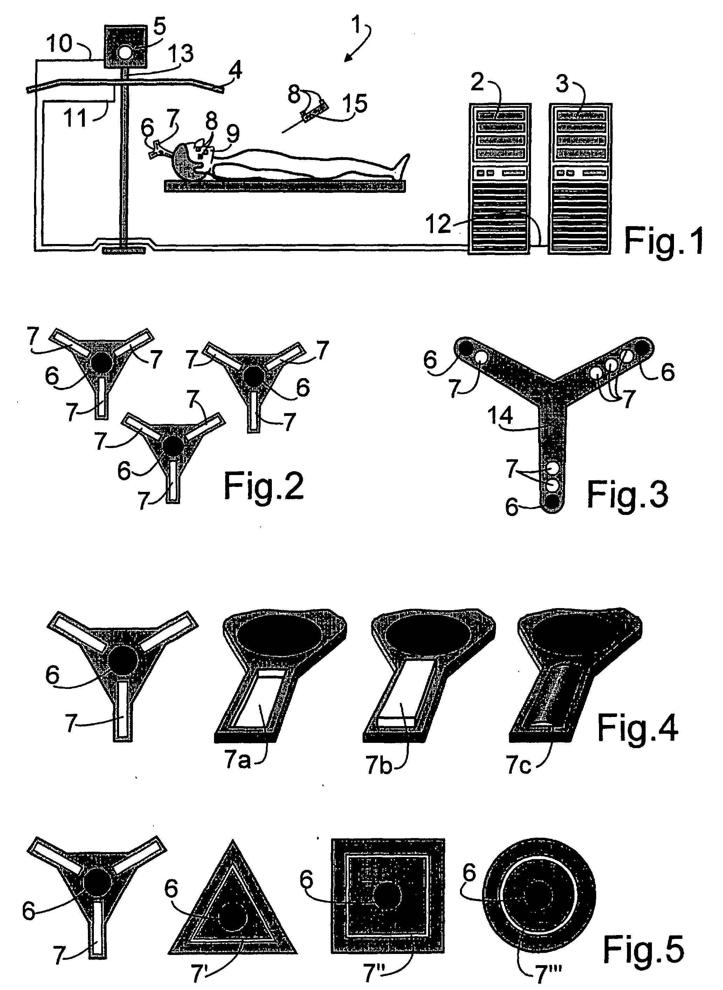

Es zeigen
- Figur 1: eine Ansicht der Vorrichtungen im Operationssaal,
- Figur 2: eine Detailansicht der 3D-Referenzmarker mit Vorrichtung zur Erfassung derselben durch optische Verfahren mittels 3D-Scanner,
- Figur 3: geometrische Vorrichtung zur optischen Erfassung von 3D-Referenzmarkern, die auf einem Rahmen fest aufgebracht sind,
- Figur 4: Perspektivische Darstellung der 3D-Referenzmarker mit zugeordneten geometrischen Vorrichtungen unterschiedlicher Form (Vertiefung/Sulcus, Leiste/Crista, plane farbcodierte Fläche) zur optischen Erfassung mit dem 3D-Scanner,
- Figur 5: alternative, beispielhaft unterschiedliche geometrische Formen mit räumlich bekannter Zuordnung zum 3D-Marker,
- Fig. 6: weitere gekoppelte Referenzierungsmarker,
- Fig. 7: Ausführungsform mit anderer Kopplung zwischen Scanner und Positionserfassungseinheit,
- Fig. 8: Referenzierung nicht notwendigerweise formstabiler Körper,
- Fig. 9: Vorrichtung zur Fixierung nicht notwendigerweise formstabiler Körper.

Die Erfindung wird nachstehend an Hand eines ersten Ausführungsbeispieles näher beschrieben.

Die Gesamtvorrichtung 1 dient der optischen Referenzierung zwischen Operationssitus, Patientendatensatz und 3D-Markern.

An einer Positionserfassungseinheit 4 ist über eine Koppel-Verbindung 13 ein optischer 3D-Scanner 5 befestigt. Die Positionserfassungseinheit 4 kann beispielsweise Infrarotsignale, Ultraschallsignale oder elektromagnetische Signale aufzeichnen und erlaubt die Bestimmung dreidimensionaler Koordinaten eines entsprechenden 3D-Markers 6 (beispielsweise: Ultraschallsender, Infrarotsender, elektromagnetischer Sender bzw. Reflektoren 17 für Wellen aller Art, Ultraschall, Infrarot, Radar etc.). Der 3D-Scanner 5 (beispielsweise ein 3D-Laser-Scanner 5 oder Radar 5a) kann die Form und Farbe von Oberflächen (beispielsweise 7 erfassen, nicht jedoch die Signale der 3D-Marker 6. Die Signale der 3D-Marker 6 können aktiv z.B. durch LED, oder passiv, z.B. durch Reflektoren, ausgesendet werden.

Die von der Positionserfassungseinheit 4 und dem 3D-Scanner 5 oder Radar 5a gemessenen Daten werden über eine Verbindung 10 und 11 an eine Anzeige- und Verarbeitungseinheit 2 weitergeleitet. Weil die Positionserfassungseinheit 4 und der 3D-Scanner 5 über eine Verbindung 13 bekannte geometrische Relationen gekoppelt bzw. kinematisch über eine Verbindung 13 aneinander befestigt sind, lassen sich auf der Verarbeitungseinheit 2 alle mit der Positionserfassungseinheit 4 ermittelten Koordinaten auch im Koordinatensystem des 3D-Scanners 5 und umgekehrt ausdrücken.

Mit der Anzeige- und Verarbeitungseinheit 2 ist über 12 eine Planungseinheit 3 verbunden. Auf dieser Planungseinheit 3 können Operationen simuliert, beispielsweise auch Umstellungsosteotomien vor einer Knochensegmentnavigation geplant werden.

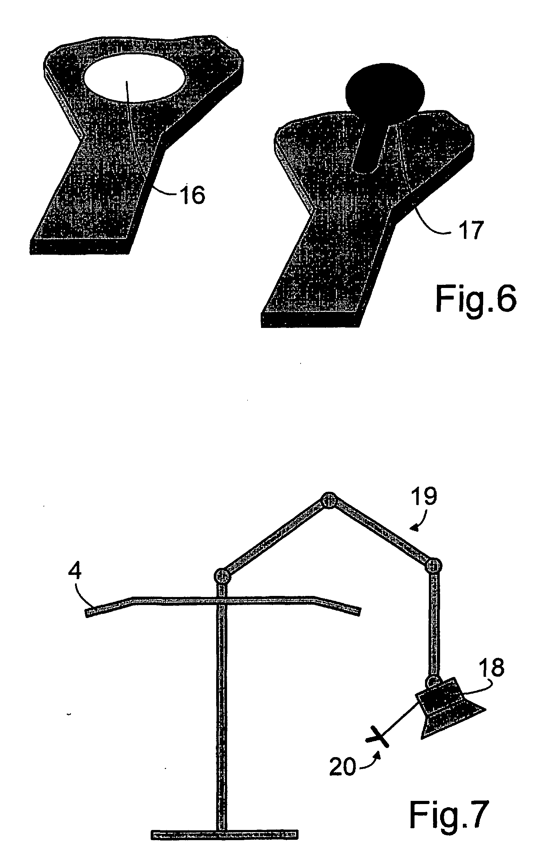

Am Patienten bei diesem Ausführungsbeispiel sind mindestens drei 3D-Marker 6 befestigt, die ein Koordinatensystem am Patienten definieren. In einer bekannten, fixen räumlichen Beziehung zu diesen 3D-Markern 6 sind geometrische Figuren 7 angebracht, die vom 3D-Scanner 5 erkannt werden können. Diese Figuren 7 können beispielsweise als eine Vertiefung/Sulcus 7a, eine erhabene Leiste/Crista 7b, als farbcodierte Linien und Felder 7c oder Strichcode bzw. Barcode ausgebildet sein. Die geometrische Figur 7 kann ebenso auch durch den Sockel, auf dem ein 3D-Marker 6 aufsitzt, gebildet werden. Die geometrische Figur 7 kann ebenso auch direkt durch einen oder mehrere 3D-Marker 6 gebildet werden.

Die Geometrie der Vorrichtungen 7 erlaubt eine eindeutige Rückrechnung der Koordinaten der 3D-Marker 6 auf der Verarbeitungseinheit 2. Die Geometrie dieser Vorrichtungen 7 kann unterschiedlich ausgebildet sein (7', 7'', 7'") , sie muß lediglich vom 3D-Scanner 5 erfaßt und von der Verarbeitungseinheit 2 zur Bestimmung der Koordinaten der 3D-Marker 6 verwendet werden können.

Sind die drei 3D-Marker 6 zur Definition eines Patientenkoordinatensystems über einen Rahmen 14 fest miteinander verbunden, dann können aus der Anordnung geometrischer Figuren 7 auf diesem Rahmen 14 die Koordinaten der 3D-Marker 6 auf der Verarbeitungseinheit 2 errechnet werden.

Der Scanner kann alternativ auch die Analyse der bekannten Geometrien der 3D-Marker direkt dazu verwenden, eine Rückrechnung der Koordinaten zu ermöglichen.

Die Referenzierung zwischen Operationssitus, Patienten-Datensatz und 3D-Markern 6 erfolgt, indem zunächst die Weichteile (vor der Operation, d.h. vor einer Weichteilschwellung oder -verlagerung) oder Knochenoberflächen 9 des Patienten mit dem 3D-Scanner 5 erfaßt werden. Auf der Verarbeitungseinheit 2 werden die Daten des 3D-Scanners 5 verrechnet und die günstigste Oberflächenpaßform zwischen Patient und Patientendatensatz bestimmt. Danach läßt sich mittels Koordinatentransformation eine Referenzierung zwischen Patient und Patientendatensatz herstellen.
Damit sind allerdings noch nicht die 3D-Marker 6 vom 3D-Scanner 5 erfaßt. Da jedoch zusammen mit dem Patient auch die geometrischen Vorrichtungen 7 um die 3D-Marker 6 mitgescannt wurden und weil die räumliche Beziehung zwischen 3D-Markern 6 und geometrischen Vorrichtungen 7 bekannt sind, lassen sich die Koordinaten der 3D-Marker 6 sowohl im Koordinatensystem der vom 3D-Scanner 5 gelieferten Daten als auch im Koordinatensystems des Patientendatensatzes abbilden.

Weitere 3D-Marker 8, die entweder direkt auf einem Knochensegment 9 oder auf einem Arbeitswerkzeug 15 aufgebracht sind oder über eine Meßkinematik oder eine Koordinatenmeßeinrichtung mit diesen gekoppelt sind, lassen sich anschließend im Patientendatensatz auf der Anzeige- und Verrechnungseinheit 2 abbilden.

Damit kann auch ein räumlicher Versatz eines Knochensegments 9, der auf der Planungseinheit 3 simuliert wurde, am Patienten reproduziert werden.

Anstelle einer Kopplung in Form einer festen Verbindung zwischen 3-D-Scanner 5 und 3D-Marker-Positionserfassungseinheit 4 ist es auch möglich, daß der 3D-Scanner 5 mit der Positionserfassungseinheit 4 als Kopplung nicht starr fixiert ist, sondern gegenüber der 3D-Marker-Positionserfassungseinheit 4 mobil bleibt und selbst mit 3D-Markern 8 ausgestattet ist, um von der 3D-Marker-Positionserfassungseinheit 4 registriert werden zu können.

Fig. 6 zeigt zeigt einen 3D-Marker 16 in einer Ausführung als LED und in der Ausführung als passive Reflektorkörper 17. Die 3D-Geometrie der Körper ist soweit bekannt, daß sie zur eindeutigen Rückrechnung der Koordinaten des Markers aus den Scanner-Daten direkt verwendet kann, ohne daß ein zusätzliche Kodierung eingebracht werden muß. Die Marker sind direkt als Vorrichtungsgeometrien geeignet.

Fig. 7 zeigt die Ausführung eines Scanners 18 mit einer als Meßform 19 ausgebildeten Koordinaten-Meßkinematik, die mit der Positionserfassungseinheit direkt verbunden ist. Über die zweite Koordinaten-Meßkinematik kann die relativ Position des Scanners 18 gegebenfalls mit erheblich höherer Genauigkeit und Meßfrequenz erfaßt werden. Alternativ dazu ist die Basis der Koordinaten-Meßkinematik selbst mit einem Lagereferenzkörper 20 ausgestattet. Im einfachsten Fall ist die Koordinaten-Meßkinematik ein einfacher Körper (z.B. Stange) mit bekannter Geometrie. Die Koordinaten-Meßkinematik kann vorteilhaft auch am Tisch oder direkt am Patienten angebracht werden, je nachdem welche Relativgenauigkeit zwischen Markern und Körper maximiert werden soll.

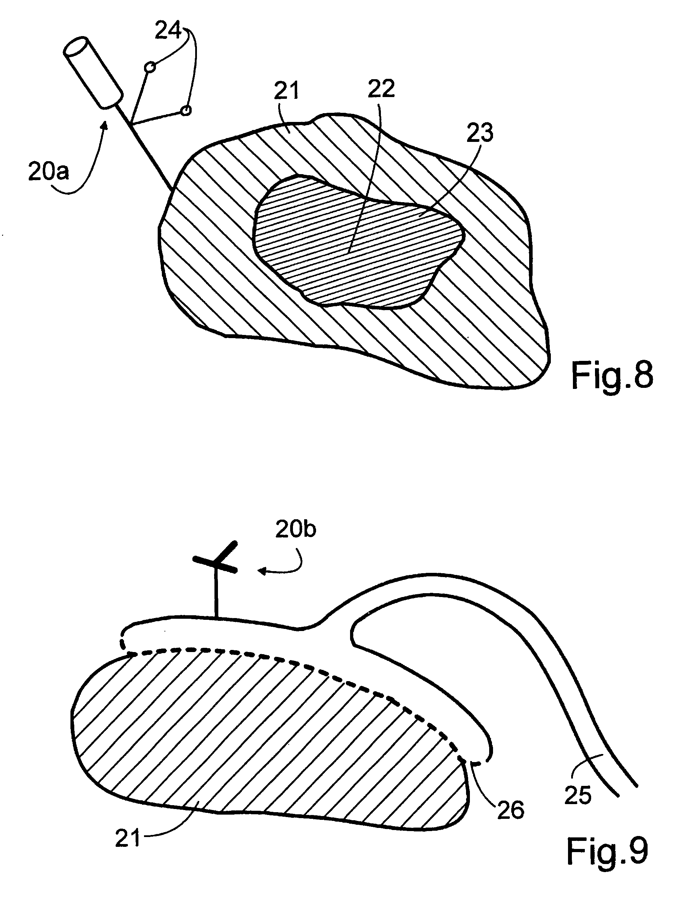

Fig. 8 zeigt anstatt eines Knochens (Hartgewebe) die verallgemeinerte Situation mit nicht notwendigerweise formstabilem Gewebe bzw. einem beliebigen Körper 21. Hier wird im einfachsten Fall eine Relation über einen Körperschwerpunkt 22 oder ein anderes Referenzvolumen 23hergestellt. Dies ist von Vorteil, wenn das Verfahren auch auf Weichgewebe, Organe oder Implantate bei Ausrichtung, Transplantation und Implantation angewendet werden soll. Auch wenn keine perfekte Formstabilität erreicht wird, kann eine Navigationshilfe stattfinden. Am Lagerreferenzkörper 20a sind Elementarkörper 24 angeordnet.

Fig. 9 zeigt eine Vorrichtung zum Fixieren der Lagereferenzkörper 20b an nicht notwendigerweise formstabilen Körpern 21. Der Lagereferenzkörper 20b ist dabei an einem Mechanismus zum befestigen nicht formstabiler Körper 21 angebracht. Im Beispiel wird über ein Unterdruckverfahren durch ein Lumen 25 und durch eine Membran 26 Körpergewebe angesaugt und in eine vorgegebene Form gepreßt. Diese Form kann wiederum vorteilhaft gestaltet werden, um beispielsweise bei der Transplantation oder Implantation das Einfügen zu erleichtern. Andere Verfahren zur Fixierung des Gewebes an der Vorrichtung wie z.B. Kleben, Kletten oder Nähen sind ebenfalls möglich.

## Patentansprüche

1. Vorrichtung zur Instrumenten- und Knochensegmentsowie Gewebe- und Organnavigation,
- wobei eine 3D-Marker-Positionserfassungseinheit (4) zur Erfassung von Signalen von 3D-Markern und ein optischer 3D-Scanner (5) oder Radar (5a) zur Erfassung von Oberfläche vorgeschen sind,
**dadurch gekennzeichnet, daß** die 3D-Marker-Positionserfassungseinheit (4) mit dem 3D-Scanner (5) oder Radar (5a) gekoppelt ist und
- daß mit den 3D-Markern (6) geometrische Figuren (7) verbunden sind oder ein oder mehrere Marker (6) als geometrische Figuren (7) ausgestaltet sind, die von dem 3D-Scanner (5) oder Radar (5a) erfaßt werden können und auf der Anzeige- und Verarbeitungseinheit (2) eine Bestimmung der Koordinaten der 3D-Marker (6) ermöglichen.

2. Vorrichtung nach Anspruch 1,
**dadurch gekennzeichnet, daß**
die Anzeige- und Verarbeitungseinheit (2)
- eine Referenzierung zwischen Operationssitus und Patientendatensatz durch eine rechnerische Flächenpassung ermöglicht und
- eine Referenzierung zwischen den Daten der 3D-Positionserfassungseinheit (4) und den Daten des 3D-Scanners (5) mittels geometrischer Vorrichtungen (7) an den 3D-Markern (6) ermöglicht.

3. Vorrichtung nach Anspruch 1,
**dadurch gekennzeichnet, daß**
bei/vor/nach Referenzierung zwischen Operationssitus, Patientendatensatz und 3D-Markern (6) weitere 3D-Marker (8) auf einem Knochensegment (9) des Patienten oder einem Instrument (15) aufgebracht sind, um eine Knochensegmentnavigation oder Instrumentennavigation zu gewährleisten.

4. Vorrichtung nach Anspruch 1,
**dadurch gekennzeichnet, daß**
geometrische Figuren (7) am oder auf dem Rahmen (14) miteinander verbundener 3D-Marker (6) aufgebracht sind.

5. Vorrichtung nach Anspruch 1,
**dadurch gekennzeichnet, daß**
ein Sockel, auf dem ein 3D-Marker (6) aufgebracht ist, als geometrische Figur (7) zur Bestimmung der Koordinaten der 3D-Marker anhand der 3D-Scanner- (5) -Meßdaten auf der Anzeige- und Verarbeitungseinheit (2) dient.

6. Vorrichtung nach Anspruch 1,
**dadurch gekennzeichnet, daß**
der Rahmen (14) miteinander verbundener 3D-Marker (6) die geometrische Figur (7) zur Bestimmung der Koordinaten der 3D-Marker anhand der 3D-Scanner- (5) -Meßdaten auf der Anzeige- und Verarbeitungseinheit (2) bildet.

7. Vorrichtung nach Anspruch 1,
**dadurch gekennzeichnet, daß**
die geometrischen Figuren (7), die vom 3D-Scanner (5) erfaßt werden können, als Vertiefung/Sulcus (7a) oder als Leiste/Crista (7b) oder als Kugel ausgebildet sind.

8. Vorrichtung nach Anspruch 1,
**dadurch gekennzeichnet, daß**
die geometrischen Figuren (7), die vom 3D-Scanner erfaßt werden können, farbcodiert oder als Strichcode oder Barcode ausgebildet sind.

9. Vorrichtungen nach Anspruch 1,
**dadurch gekennzeichnet, daß**
unterschiedliche geometrische Figuren (7', 7'' 7''') die vom 3D-Scanner (5) erfaßt werden Können, mit 3D-Markern (6) verbunden sind.

10. Vorrichtungen nach Anspruch 1,
**dadurch gekennzeichnet, daß**
die Kopplung zwischen 3D-Marker-Positionserfassungseinheit (4) und 3D-Scanner (5) oder Radar (5a) eine feste Verbindung ist.

11. Vorrichtungen nach Anspruch 1,
**dadurch gekennzeichnet, daß**
die Kopplung zwischen 3D-Marker-Positionserfassungseinheit (4) und 3D-Scanner (5) oder Radar (5a) eine Verbindung über einen Koordinaten-Meßarm (19) ist.

12. Vorrichtung nach Anspruch 1,
**dadurch gekennzeichnet, daß**
der 3D-Scanner (5) mit der Positionserfassungseinheit (4) als Kopplung nicht starr fixiert ist, sondern gegenüber der 3D-Marker-Positionserfassungseinheit (4) mobil bleibt und selbst mit 3D-Markern (8) ausgestattet ist, um von der 3D-Marker-Positionserfassungseinheit (4) registriert werden zu können.

13. Vorrichtung nach Anspruch 12,
**dadurch gekennzeichnet, daß**
der 3D-Scanner als ein hand-held-3D-Scanner ausbildet ist.

14. Vorrichtung nach Anspruch 1,
**dadurch gekennzeichnet, dass**
der 3D-Scanner (5) und die Positionserfassungseinheit (4) dieselben Empfängerkomponenten zur Erfassung der Positionsinformation und der 3D-Information verwenden.

15. Vorrichtung nach Anspruch 14,
**dadurch gekennzeichnet, dass**
der 3D-Scanner (5) und die Positionserfassungseinheit (4) dieselben Senderkomponenten zur Erfassung der Positionsinformation und 3D-Information verwenden bzw. dieselben physikalischen Senderwellen auswerten.

## Claims

1. Device for instrument and bone segment as well as tissue and organ navigation,
wherein a 3-D marker position detection unit (4) for detecting signals from 3-D markers and an optical 3-D scanner (5) or radar unit (5a) for detecting surfaces are provided, **characterized in**
- **that** the 3-D marker position detection unit (4) is coupled to the 3-D scanner (5) or radar unit (5a) and
- **that** geometric figures (7) are connected with the 3-D markers (6) or one or several markers (6) are shaped as geometric figures (7) that can be detected by the 3-D scanner (5) or radar unit (5a), thereby allowing a determination of the coordinates of the 3-D markers (6) on the display and processing unit (2).

2. Device according to claim 1,
**characterized in that**
the display and processing unit (2)
- enables referencing between an operating site and a patient data set through a computational surface fit and
- enables referencing between the data of the 3-D position detection unit (4) and the data of the 3-D scanner (5) with the help of geometric devices (7) disposed on the 3-D markers (6).

3. Device according to claim 1,
**characterized in that**
during/before/after referencing between the operating site, the patient data set and the 3-D markers (6), additional 3-D markers (8) are applied on a bone segment (9) of the patient or on an instrument (15) for the purpose of enabling a bone segment navigation or instrument navigation.

4. Device according to claim 1,
**characterized in that**
geometric figures (7) are applied to or on the frame (14) of 3-D markers (6) that are connected with each other.

5. Device according to claim 1,
**characterized in that**
a base, on which a 3-D marker (6) is placed, is provided as a geometric figure (7) for determining the coordinates of the 3-D markers on the display and processing unit (2) from the 3-D scanner (5) measurement data.

6. Device according to claim 1,
**characterized in that**
the frame (14) of 3-D markers (6) that are connected with each other, forms the geometric figure (7) for determining the coordinates of the 3-D markers on the display and processing unit (2) from the 3-D scanner (5) measurement data.

7. Device according to claim 1,
**characterized in that**
that geometric figures (7) that can be detected by the 3-D scanner (5) are formed as a recess/sulcus (7a) or as a raised portion/crista (7b) or as a sphere.

8. Device according to claim 1,
**characterized in that**
the geometric figures (7) that can be detected by the 3-D scanner, are color-coded or formed as a bar code.

9. Devices according to claim 1,
**characterized in that**
different geometric figures (7', 7", 7"') that can be detected by the 3-D scanner (5), are connected with 3-D markers (6).

10. Devices according to claim 1,
**characterized in that**
the coupling between the 3-D marker position detection unit (4) and the 3-D scanner (5) or the radar unit (5a) is a fixed connection.

11. Devices according to claim 1,
**characterized in that**
the coupling between the 3-D marker position detection unit (4) and the 3-D scanner (5) or the radar unit (5a) is a connection via a coordinate measuring arm (19).

12. Device according to claim 1,
**characterized in that**
the 3-D scanner (5) is not rigidly coupled with the position detection unit (4), but remain mobile with respect to the 3-D marker position detection unit (4) and is itself provided with 3-D markers (8) so as to be able to be registered by the 3-D marker position detection unit (4).

13. Device according to claim 12,
**characterized in that**
the 3-D scanner is implemented as a hand-held 3-D scanner.

14. Device according to claim 1,
**characterized in that**
the 3-D scanner (5) and the position detection unit (4) use the same receiver components for detecting the position information and the 3-D information.

15. Device according to claim 14,
**characterized in that**
the 3-D scanner (5) and the position detection unit (4) use the same transmitter components for detecting the position information and the 3-D information and/or process the same physical transmitter waves.

## Revendications

1. Dispositif pour la navigation d'instrument, de segment osseux, de tissu et d'organe,
- où sont prévus une unité de saisie de position (4) à marqueurs 3 D pour la saisie de signaux de marqueurs 3 D et un scanner optique 3 D (5) ou radar (5a) pour la saisie de surfaces
**caractérisé en ce que**
l'unité de saisie de position (4) à marqueurs 3 D est couplée au scanner 3 D (5) ou au radar (5a) et **en ce que** des figures géométriques (7) sont reliées au marqueurs 3 D (6) ou **en ce qu'**un ou plusieurs marqueurs (6) sont formés comme figures géométriques (7), pouvant être saisies par le scanner 3 D (5) ou le radar (5a) et rendant possible une définition des coordonnées des marqueurs 3 D (6) sur l'unité d'affichage et de traitement (2).

2. Dispositif selon la revendication 1,
**caractérisé en ce que**
l'unité d'affichage et de traitement (2)
- permet un référencement entre le site opératoire et l'enregistrement du patient par un ajustement théorique de surface
- permet un référencement entre les données de l'unité de saisie de position 3 D (4) et les données du scanner 3 D (5) au moyen de dispositifs géométriques (7) sur les marqueurs 3 D (6).

3. Dispositif selon la revendication 1,
**caractérisé en ce que**
pendant le référencement/avant le référencement/après le référencement entre le site opératoire, l'enregistrement du patient et les marqueurs 3 D (6), d'autres marqueurs 3 D (8) sont appliqués sur un segment osseux (9) du patient ou sur un instrument (15), pour assurer une navigation de segment osseux ou d'instrument.

4. Dispositif selon la revendication 1,
**caractérisé en ce que**
des figures géométriques (7) sont appliquées contre ou sur le cadre (14) des marqueurs 3 D (6) reliés entre eux.

5. Dispositif selon la revendication 1,
**caractérisé en ce qu'**
un socle sur lequel est appliqué un marqueur 3 D (6) sert de figure géométrique (7) pour la définition des coordonnées des marqueurs 3 D sur l'unité d'affichage et de traitement (2) à partir des données de mesure du scanner 3 D (5).

6. Dispositif selon la revendication 1,
**caractérisé en ce que**
le cadre (14) des marqueurs 3 D (6) reliés entre eux forme la figure géométrique (7) pour la définition des coordonnées des marqueurs 3 D sur l'unité d'affichage et de traitement (2) à partir des données de mesure du scanner 3 D (5).

7. Dispositif selon la revendication 1,
**caractérisé en ce que**
les figures géométriques (7) pouvant être saisies par le scanner 3 D (5) sont formées comme évidement/gouttière (7a) ou comme crête/saillie (7b) ou comme sphère.

8. Dispositif selon la revendication 1,
**caractérisé en ce que**
les figures géométriques (7) pouvant être saisies par le scanner 3 D sont codées par couleurs ou comme codes optiques ou codes-barres.

9. Dispositifs selon la revendication 1,
**caractérisés en ce que**
différentes figures géométriques (7', 7", 7''') pouvant être saisies par le scanner 3 D (5) sont reliés à des marqueurs 3 D (6).

10. Dispositifs selon la revendication 1,
**caractérisés en ce que**
le couplage entre l'unité de saisie de position (4) à marqueurs 3 D et le scanner 3 D (5) ou le radar (5a) est une connexion fixe.

11. Dispositifs selon la revendication 1,
**caractérisés en ce que**
le couplage entre l'unité de saisie de position (4) à marqueurs 3 D et le scanner 3 D (5) ou le radar (5a) est une connexion par un bras de mesure des coordonnées (19).

12. Dispositif selon la revendication 1,
**caractérisé en ce que**
le scanner 3 D (5) n'est pas rigidement fixé en tant que couplage avec l'unité de saisie de position (4), mais reste mobile par rapport à l'unité de saisie de position (4) à marqueurs 3 D et est lui-même pourvu de marqueurs 3 D (8) afin de pouvoir être enregistré par l'unité de saisie de position (4) à marqueurs 3 D.

13. Dispositif selon la revendication 12,
**caractérisé en ce que**
le scanner 3 D est formé comme scanner 3 D portatif.

14. Dispositif selon la revendication 1,
**caractérisé en ce que**
le scanner 3 D (5) et l'unité de saisie de position (4) utilisent les mêmes composants récepteurs pour la saisie de l'information de position et de l'information 3 D.

15. Dispositif selon la revendication 14,
**caractérisé en ce que**
le scanner 3 D (5) et l'unité de saisie de position (4) utilisent les mêmes composants émetteurs pour la saisie de l'information de position et de l'information 3 D, ou évaluent les mêmes ondes physiques d'émetteur.
